Europäisches Patentamt

(19) European Patent Office

Office européen des brevets

(11) Numéro de publication: **0 176 457**
**B1**

(12) **FASCICULE DE BREVET EUROPEEN**

(45) Date de publication du fascicule du brevet:
**01.04.87**

(21) Numéro de dépôt: **85420166.2**

(22) Date de dépôt: **13.09.85**

(51) Int. Cl.⁴: **C 07 C 11/04,** C 07 C 1/36

(54) Procédé de fabrication d'éthylène à partir d'esters éthyliques.

(30) Priorité: **17.09.84 FR 8414399**

(43) Date de publication de la demande:
**02.04.86 Bulletin 86/14**

(45) Mention de la délivrance du brevet:
**01.04.87 Bulletin 87/14**

(84) Etats contractants désignés:
**BE DE FR GB IT NL SE**

(56) Documents cités:
**US - A - 4 270 015**
**US - A - 4 399 305**

(73) Titulaire: **ATOCHEM, 4 & 8, Cours Michelet La Défense 10, F-92800 Puteaux (FR)**

(72) Inventeur: **Cognion, Jean-Marie, 85, route de Charly, F-69230 St.-Genis Laval (FR)**
Inventeur: **Duruai, Pierre, "Les Essarts No.2" Chemin du Rossignol, F-69390 Vernaison (FR)**

ACTORUM AG

## Description

La présente invention concerne la production d'éthylène par décomposition catalytique des esters éthyliques.

L'éthylène est actuellement produit essentiellement par craquage à la vapeur d'eau des coupes légères de pétrole.

La raréfaction du pétrole et l'augmentation de son coût font que l'industrie cherche à diversifier les voies d'accès à l'éthylène pour satisfaire économiquement la demande croissante en ce produit, en particulier en qualité haute pureté.

La synthèse directe par une réaction de FISCHER TROPSCH à partir du mélange $CO-H_2$ qu'est le gaz de synthèse préparé à partir de charbon ou de gaz naturel, est malheureusement non sélective.

La déshydratation de l'éthanol, produit de fermentation de végétaux ou de sucre, ne conduit sélectivement à l'éthylène qu'à un coût encore prohibitif.

La condensation catalytique du méthanol, particulièrement étudiée sur des zéolithes synthétiques par la Société MOBIL OIL CORPORATION et qui met en jeu une matière première facilement accessible à partir du gaz de synthèse, n'a pas permis de concilier sélectivité et productivité.

Une autre voie d'accès à l'éthylène est la décomposition thermique, ou pyrolyse, des esters éthyliques. Cette pyrolyse est connue depuis longtemps et elle s'effectue à des températures pouvant atteindre 500 à 600° C.

Par exemple, R.F. MAKENS et W.G. EVERSOLE décrivent dans J. Am. Chem. Soc. 61, 1939, 3203, la pyrolyse du formiate d'éthyle à une température de 375° C au moins. L'éthylène formé est non seulement accompagné de produits de décomposition de l'acide formique, mais encore d'une quantité importante d'hydrocarbures tels que le méthane, l'éthane et le butylène.

Le brevet des Etats-Unis d'Amérique N° 4 270 015 décrit l'obtention d'esters éthyliques par réaction catalytique d'un acide carboxylique aliphatique renfermant 2 à 4 atomes de carbone avec du gaz de synthèse, puis celle d'éthylène et de l'acide dont dérive l'ester, par pyrolyse de ce dernier dans un réacteur en quartz à une température de l'ordre pratiquement de 450° C.

L'éthylène produit contient d'autres hydrocarbures, particulièrement l'éthane, comme il est encore rapporté par John F. KNIFTON dans Journal of Catalysis 79, 147-155 (1983).

La concentration d'éthane peut atteindre des valeurs élevées, près de 5% en pyrolysant le propionate d'éthyle, pourtant pur, à 460° C.

De plus, la conversion de l'ester et la productivité en éthylène sont faibles.

Pour tenter de remédier aux inconvénients de la pyrolyse non catalytique des esters éthyliques, il a été proposé d'en assurer la décomposition catalytique.

Le brevet des Etats-Unis d'Amérique N° 4 399 305 décrit l'obtention d'éthylène de haute pureté à partir d'acétate d'éthyle à l'aide d'un catalyseur constitué d'une résine perfluorosulfonique commercialisée sous le nom de NAFION® par la Société DUPONT de NEMOURS.

Si la portion gazeuse du produit recueilli après pyrolyse est dite être de l'éthylène haute pureté, la productivité du procédé exposé reste faible par suite d'une conversion de l'ester ne dépassant pas 20% dans le mode de réalisation de l'invention pourtant le meilleur. De plus il n'est fait aucune mention de la sélectivité.

L'art antérieur enseigne encore que l'emploi de catalyseurs à base d'alumine ou de silice-alumine ne permet pas de satisfaire les exigences actuelles de l'industrie.

Par exemple, l'acétate d'éthyle conduit à un éthylène fortement pollué par du $CO_2$ lorsque, selon SENDERENS, Bull. Soc. Chim. France, 1908, 4, N° 3, p. 826, il est décomposé à 300° C sur alumine et lorsque, selon R.D. OBOLENTSEV et Yu. N. USOV. Doklady Akad. Nauk. SSSR, 71, 1950, 489, il est décomposé à 400° C sur silice-alumine.

Le procédé selon la présente invention autorise l'obtention d'éthylène haute pureté ou d'un mélange éthylène-monoxyde de carbone directement utilisable, en même temps qu'il permet d'accéder à une forte conversion de l'ester, à une sélectivité et à une productivité élevées.

Il consiste à effectuer en phase gazeuse la décomposition d'esters éthyliques d'acides carboxyliques aliphatiques, à une température comprise entre 150° C et 300° C, en présence d'un catalyseur choisi parmi les zéolithes dont le diamètre de pore est au moins de 0,6 nm.

Parmi de telles zéolithes sont employées de préférence les mordénites, les zéolithes X et les zéolithes Y.

La préparation des mordénites à larges pores est par exemple décrite dans le brevet des Etats-Unis d'Amérique N° 4 018 514 et dans Mol. Sieves Pap. Conf., 1967, 78, Soc. Chem. Ind. London, par D. DOMINE et J. QUOBEX.

La zéolithe X est décrite par exemple dans le brevet des Etats-Unis d'Amérique N° 2 882 244, la zéolithe Y dans le brevet des Etats-Unis d'Amérique N° 3 130 007.

Les zéolithes convenant au procédé de l'invention peuvent être mises en œuvre sous la forme basique, sous la forme partiellement ou totalement acidifiée, ou sous la forme partiellement désaluminée.

Elles peuvent être mises en œuvre par exemple sous forme de billes ou de cylindres de diamètre le plus souvent compris entre 1 et 5 mm, obtenus lorsque la zéolithe est fabriquée à l'état de poudre, en appliquant à celle-ci les procédés classiques d'agglomération, d'extrusion, de pastillage.

Le procédé a lieu à une pression proche de la pression atmosphérique suffisante pour vaincre la perte de charge du lit catalytique.

Bien qu'il puisse être conduit à des températures non comprises dans la gamme 150° C-300° C, une température inférieure à 150° C est cause d'une vitesse de décomposition de l'ester trop lente pour rester compatible avec en particulier les exigences industrielles de productivité, une température supérieure à 300° C entraînant pour sa part une baisse de la sélectivité et un risque d'encrassement du catalyseur.

La gamme de température ainsi définie ci-dessus convient particulièrement bien pour la décomposition des esters éthyliques des acides formique, acétique et propionique, qui, seuls ou en mélange, sont préférés.

L'ester à décomposer est vaporisé préalablement à son passage sur le catalyseur. Il peut être introduit seul dans le réacteur de catalyse mais aussi sous forme d'un mélange gazeux avec un inerte tel que par l'exemple l'azote.

Le temps de séjour sur le catalyseur peut varier dans de larges limites compte tenu en particulier de la nature du catalyseur et de l'ester. Il est le plus généralement compris entre 10 et 100 secondes.

Les exemples suivants, donnés à titre indicatif mais non limitatif, illustrent le procédé de l'invention. La pression, pour chacun d'eux, est la pression proche de la pression atmosphérique suffisante pour vaincre la perte de charge du lit catalytique.

*Exemple 1:*

Dans un réacteur tubulaire en verre pyrex de 30 mm de diamètre intérieur pouvant être porté à température contrôlée, sont disposés 30 ml de pastilles cylindriques de 5 mm de diamètre et de hauteur, obtenues par agglomération mécanique d'une mordénite en poudre fabriquée par la Société Chimique de la Grande Paroisse sous la référence Alite 180.HZ.

Un mélange gazeux formé d'azote et de formiate d'éthyle contenant 70,7% d'ester en volume est introduit dans le réacteur où la décomposition du formiate d'éthyle est effectuée à une température de 250° C.

La conversion du formiate d'éthyle est égale à 100%.

La sélectivité en éthylène est de plus de 98%, moins de 2% du formiate d'éthyle engagé étant transformé en éthane.

L'éthylène est obtenu en mélange avec le monoxyde de carbone CO formé avec une sélectivité de 100% à partir du formiate d'éthyle.

*Exemple 2:*

Il est procédé, dans l'appareillage de l'exemple 1, à la décomposition du propionate d'éthyle par passage à une température de 250° C, de 3,36 l/h d'un mélange gazeux $N_2$-propionate d'éthyle renfermant 61,3% en volume d'ester, sur 30 ml du catalyseur de l'exemple 1.

20% de l'ester sont convertis avec des sélectivités en éthylène et acide propionique qui sont, dans les deux cas, supérieures à 98%.

*Exemple 3:*

En opérant à 150° C, avec 30 ml du catalyseur de l'exemple 1 et 3,8 l/h d'un mélange $N_2$-acétate d'éthyle à l'état gazeux contenant 65,8% en volume d'ester, la conversion de ce dernier en éthylène et acide acétique est égale à 5,5%, la sélectivité en éthylène aussi bien qu'en acide acétique étant de 100%.

*Exemple 4:*

En opérant comme dans l'exemple 3 mais à 250° C et sans admission d'azote, la conversion de l'ester est de 40%, la sélectivité en acide acétique de 100%, la pureté de l'éthylène recueilli est supérieure à 99,9%, seules des traces de méthane ayant été décelées.

*Exemple 5:*

30 ml d'extrudés de 3,2 mm environ de diamètre et constitués d'une mordénite commercialisée sous le nom de ZEOLON 900 Na par la Société NORTON, sont disposés dans le réacteur de l'exemple 1.

3,67 l/h d'un mélange $N_2$-acétate d'éthyle à l'état gazeux contenant 64,6% en volume d'ester sont passés sur le catalyseur à 250° C.

La conversion de l'ester est de 15%. L'éthylène et l'acide acétique sont obtenus avec une sélectivité de pratiquement 100%, l'éthylène obtenu contenant moins de 0,2% de méthane.

*Exemple 6:*

Le catalyseur de l'exemple 5 est remplacé par un volume égal d'extrudés de même dimension de mordénite ZEOLON 900 H commercialisée par la Société NORTON.

3,69 l/h d'un mélange gazeux $N_2$-acétate d'éthyle contenant 66,7% en volume d'ester sont passés sur le catalyseur à 300° C.

Au cours de la décomposition catalytique, 55% de l'ester sont convertis, la sélectivité en acétique atteignant 99% et l'éthylène produit ayant une pureté supérieure à 99,5%.

*Exemple 7:*

Sur 30 ml de zéolithe 13X sous la forme de sphères de 1,4 mm à 2,4 mm de diamètre, approvisionnée auprès de la Société Touzart et Matignon, sont passés à 300° C 3,85 l/h d'un mélange gazeux d'azote et d'acétate d'éthyle renfermant 66,3% en volume d'ester.

13% de ce dernier sont transformés avec une sélectivité de 100% en éthylène et en acide acétique.

L'éthylène produit ne contient qu'à l'état de traces du méthane et des hydrocarbures contenant trois atomes de carbone.

*Exemple 8:*

Le catalyseur de l'exemple 7 est soumis à un lavage à l'aide d'une solution de nitrate d'ammonium suivi d'un traitement thermique à 550° C.

30 ml de la zéolithe 13X ainsi partiellement acidifiée servent à la décomposition à 250° C de l'acétate d'éthyle contenu à la concentration de 63,4% en volume dans les 3,96 l/h de mélange gazeux $N_2$-ester passant sur le catalyseur.

11% de l'ester sont convertis sélectivement en éthylène et acide acétique, l'éthylène produit ne contenant que des traces de méthane.

*Exemple 9:*

Un mélange gazeux N₂-acétate d'éthyle contenant l'ester à une concentration de 61,9% en volume sont passés à raison de 3,8 l/h, à une température de 280° C, sur 30 ml de zéolithe Y de la Société ALFA référencée LZY 82 sous forme d'extrudés de 1,6 mm environ de diamètre.

Dans ces conditions 70% de l'ester sont convertis en éthylène et acide acétique avec une sélectivité pratiquement de 100%.

L'éthylène recueilli ne renferme en effet que des traces de méthane.

## Revendications

1. Procédé de fabrication d'éthylène de haute pureté par décomposition catalytique en phase gazeuse d'esters éthyliques d'acides carboxyliques aliphatiques, caractérisé en ce que ladite décomposition est effectuée à une température comprise entre 150° C et 300° C en présence d'un catalyseur choisi parmi les zéolithes dont le diamètre de pore est au moins de 0,6 nm.

2. Procédé selon la revendication 1, caractérisé en ce que les esters décomposés sont ceux des acides formique, acétique et propionique.

3. Procédé selon l'une des revendications 1 et 2, caractérisé en ce que le catalyseur est une mordénite, une zéolithe 13X ou une zéolithe Y.

4. Procédé selon l'une des revendications 1 à 3, caractérisé en ce que la zéolithe est sous forme basique ou sous forme partiellement ou totalement acidifiée ou sous forme partiellement désaluminée.

5. Procédé selon l'une des revendications 1 à 4, caractérisé en ce que la zéolithe est mise en œuvre sous forme de billes, d'extrudés ou de pastilles.

6. Procédé selon la revendication 5, caractérisé en ce que les billes, les extrudés ou les pastilles ont une dimension comprise entre 1 et 5 mm.

7. Procédé selon l'une des revendications 1 à 6, caractérisé en ce que la décomposition catalytique de l'ester est réalisée à la pression proche de la pression atmosphérique suffisante pour vaincre la perte de charge du lit catalytique.

8. Procédé selon l'une des revendications 1 à 7, caractérisé en ce que le temps de séjour sur le catalyseur est compris entre 10 et 100 secondes.

## Patentansprüche

1. Verfahren zur Herstellung von Ethylen hoher Reinheit durch katalytische Zersetzung von Ethylestern aliphatischer Carbonsäuren in der Gasphase, dadurch gekennzeichnet, dass die Zersetzung bei einer Temperatur zwischen 150° C und 300° C in Gegenwart eines Katalysators durchgeführt wird, der unter den Zeolithen ausgewählt wird, deren Porendurchmesser mindestens 0,6 nm beträgt.

2. Verfahren nach Anspruch 1, dadurch gekennzeichnet, dass die eingesetzten Ester die der Ameisen-, Essig- und Propionsäure sind.

3. Verfahren nach Anspruch 1 oder 2, dadurch gekennzeichnet, dass der Katalysator ein Mordenit, ein Zeolith 13X oder ein Zeolith Y ist.

4. Verfahren nach einem der Ansprüche 1 bis 3, dadurch gekennzeichnet, dass der Zeolith in basischer, in teilweise oder vollständig saurer oder in teilweise dealuminierter Form vorliegt.

5. Verfahren nach einem der Ansprüche 1 bis 4, dadurch gekennzeichnet, dass der Zeolith in Form von Kugeln, Extrudaten oder Pastillen eingesetzt wird.

6. Verfahren nach Anspruch 5, dadurch gekennzeichnet, dass die Kugeln, Extrudate oder Pastillen eine Grösse zwischen 1 und 5 mm besitzen.

7. Verfahren nach einem der Ansprüche 1 bis 6, dadurch gekennzeichnet, dass die katalytische Esterzersetzung bei einem Druck nahe dem atmosphärischen Druck durchgeführt wird, der ausreichend ist, den Chargenverlust des katalytischen Betts zu überwinden.

8. Verfahren nach einem der Ansprüche 1 bis 7, dadurch gekennzeichnet, dass die Verweilzeit auf dem Katalysator zwischen 10 und 100 Sekunden beträgt.

## Claims

1. Process for manufacturing ethylene of high purity by catalytic decomposition in the gaseous phase of ethyl esters of aliphatic carboxylic acids, characterized in that the said decomposition is performed at a temperature of between 150° C and 300° C in the presence of a catalyst chosen from zeolites, the pore diameter of which is at least 0.6 nm.

2. Process according to Claim 1, characterized in that the esters decomposed are those of formic, acetic and propionic acids.

3. Process according to one of Claims 1 and 2, characterized in that the catalyst is a mordenite, a zeolite 13X or a zeolite Y.

4. Process according to one of Claims 1 to 3, characterized in that the zeolite is in basic form or in partially or completely acidified form or in partially dealuminified form.

5. Process according to one of Claims 1 to 4, characterized in that the zeolite is used in the form of beads, extrudates or pellets.

6. Process according to Claim 5, characterized in that the beads, extrudates or pellets have a size of between 1 and 5 mm.

7. Process according to one of Claims 1 to 6, characterized in that the catalytic decomposition of the ester is carried out at the pressure close to atmospheric pressure which is sufficient to overcome the pressure drop of the catalytic bed.

8. Process according to one of Claims 1 to 7, characterized in that the residence time on the catalyst is between 10 and 100 seconds.